# EUROPEAN PATENT APPLICATION

(11) **EP 4 339 190 A1**
(43) Date of publication of application: **20.03.2024**
(21) Application number: 21942440.5
(22) Date of filing: 28.05.2021
(51) Int. Cl.: C07D 291/06, B01J 31/26, B01J 23/04

(54) **METHOD FOR PREPARING ACESULFAME POTASSIUM**

(71) Applicant: Anhui Jinhe Industrial Co., Ltd., Chuzhou, Anhui 239200 (CN)
(72) Inventor: ZHOU, Rui, Chuzhou, Anhui 239200 (CN); DING, Zhen, Chuzhou, Anhui 239200 (CN); CHEN, Yongxu, Chuzhou, Anhui 239200 (CN); YANG, Fengbao, Chuzhou, Anhui 239200 (CN); LIU, Gang, Chuzhou, Anhui 239200 (CN)
(74) Representative: Ipside
(86) International application number: PCT/CN2021/097017
(87) International publication number: WO 2022/246869

(57) **Abstract**

Described herein is a method for preparing acesulfame potassium, comprising: pressing an acetoacetamide-N-sulfonic acid triethylamine salt solution and a cyclizing agent solution into a Venturireactor via different inlets, mixing same at a mixing section and a diffusion section of the Venturireactor, and then injecting the obtained mixture into a flow reactor, wherein the cyclizing agent solution is formed by dissolving sulfur trioxide in a first organic solvent; subjecting the mixture passing through the flow reactor to a sulfonation cyclization reaction under the action of a supported solid base heterogeneous catalyst preset in the flow reactor, and entering the obtained sulfonated cyclized product into a hydrolysis reactor; subjecting the sulfonated cyclized product and a hydrolysis agent preset in the hydrolysis reactor to a hydrolysis reaction to obtain a hydrolysis product solution; and adding potassium hydroxide into an organic phase of the hydrolysate solution for a salt-forming reaction to obtain the acesulfame potassium.

## Description

### Technical Field

The present disclosure belongs to the technical field of fine chemical engineering, and specifically relates to a method for preparing acesulfame potassium.

### Background of the Invention

Acesulfame potassium (acesulfame), also known as AK sugar, is a widely used sugar substitute food additive and has white crystalline powder appearance. It is a kind of organic synthetic salt, with a taste similar to sugarcane, easily soluble in water, slightly soluble in alcohol, stable in chemical properties, and not prone to decomposition failure; it does not participate in body metabolism, and does not provide energy; it has high sweetness, and the price is low; it does not cause dental caries; and it is stable to heat and acid.

At present, in the synthesis of acesulfame, a diketene-sulfur trioxide method is generally used, and the specific reacting steps thereof comprise: reacting sulfamic acid with an amine to form an amino sulfamate, and then reacting the amino sulfamate with a diketene to form an acetyl acetamide salt; subjecting the acetyl acetamide salt to a cyclization reaction in the presence of sulfur trioxide to form a cyclic sulfur trioxide adduct; hydrolyzing the cyclic compound to obtain a hydrolysate (ASH); and then treating the hydrolysate with potassium hydroxide to obtain acesulfame potassium (ASK).

In the step of the sulfonation cyclization reaction, the acetyl acetamide salt and sulfur trioxide are usually directly added to the reaction kettle for reaction, the reaction efficiency thereof is not high, it also causes some impurities with structure similar to acesulfame potassium to remain in the final product acesulfame, and the yield of the reaction cannot reach a satisfying level.

In the prior art, although some improved technical methods for the sulfonation cyclization reaction are disclosed, such as Chinese patent CN103613566 B, Chinese patent CN111511724 A and Chinese patent CN112110876 A, these techniques still cannot effectively improve the conversion rate of the reaction, and also cannot solve the problem that the product (ASH) obtained after the hydrolysis of the cyclized cyclization product has lots of impurities.

### Summary of the Invention

In view of the above-mentioned problems, the present application provides a method for preparing acesulfame potassium which overcomes the above-mentioned problems or at least partially solves the above-mentioned problems.

According to a first aspect of the present application, a method for preparing acesulfame potassium is provided, the method is implemented by using a combined reactor, the combined reactor comprises a Venturi reactor, a flow reactor and a hydrolysis reactor, the outlet of the diffusion section of the Venturi reactor is connected to the inlet of the flow reactor, and the flow reactor is connected to the inlet of the hydrolysis reactor; and the method comprises:
a mixing step: pressing an acetylacetamide-N-sulfonic acid triethylamine salt solution and a cyclizing agent solution from different inlets of the Venturi reactor, and after mixing in the mixing section and the diffusion section of the Venturi reactor, injecting the obtained mixture into the flow reactor, wherein the cyclizing agent solution is formed by dissolving sulfur trioxide in a first organic solvent;
a sulfonation cyclization step: subjecting the mixture passing through the flow reactor to a sulfonation cyclization reaction under the action of a supported solid base heterogeneous catalyst preset in the flow reactor, and entering the obtained sulfonated cyclized product into the hydrolysis reactor;
a hydrolysis step: subjecting the sulfonated cyclized product to a hydrolysis reaction with a hydrolysis agent preset in the hydrolysis reactor to obtain a hydrolysate solution; and
a salt forming step: adding potassium hydroxide into the organic phase of the hydrolysate solution, and carrying out a salt forming reaction to obtain acesulfame potassium.

Optionally, in the above method, the acetylacetamide-N-sulfonic acid triethylamine salt solution is prepared by the following method:
dissolving sulfamic acid in a second organic solvent, then adding triethylamine thereinto, and carrying out an amination reaction to produce an ammonium sulfamate solution; and
adding diketene to the obtained ammonium sulfamate solution, and carrying out an acylation reaction to obtain an acetylacetamide-N-sulfonic acid triethylamine salt solution.

Optionally, in the above method, the molar ratio of sulfamic acid to the second organic solvent is 1: 3-5, preferably 1: 4;
the molar ratio of diketene to sulfur trioxide is 1: 2-15, preferably 1: 4;
the reaction temperature of the sulfonation cyclization reaction is -20 to 40° C, preferably 10 to 30° C, and more preferably 15 to 25° C; and the reaction time is 1 s to 30 min, preferably 2 s to 2 min.

Optionally, in the above method, the first organic solvent is pentane, hexane, heptane or dichloromethane; and
the second organic solvent is pentane, hexane, heptane or dichloromethane.

Optionally, in the above method, the supported solid base heterogeneous catalyst is a K₂O/-γAl₂O₃ catalyst, and preferably, organic silicone is added to the K₂O/-γAl₂O₃ catalyst.

Optionally, in the above method, the hydrolysis agent is an ethanol aqueous solution, wherein the mass fraction of ethanol in the ethanol aqueous solution is 60-85%.

Optionally, in the above method, the reaction temperature of the hydrolysis reaction is -10 to -20° C.

Optionally, in the above method, using the acetylacetamide-N-sulfonic acid triethylamine salt solution as a working fluid, pressing the working fluid from the nozzle of the Venturi reactor, using the cyclizing agent solution as an ejecting fluid, and pressing the ejecting fluid into the suction chamber of the Venturi reactor; and controlling the pressure of the working fluid to be greater than the pressure of the ejecting fluid.

Optionally, in the above method, the pressure of the working fluid is 0.4-1.6 MPa, the pressure of the ejecting fluid is 0.2-1.4 MPa, and the pressure of the working fluid is 0.2-0.4 MPa greater than the pressure of the ejecting fluid.

Optionally, in the above method, the flow reactor is a fixed bed reactor or a tubular reactor.

The beneficial effects of the present application lie in that the Venturi reactor, the flow reactor and the hydrolysis reactor are combined for use to form a set of combined continuous reactor. In the combined reactor, an acetylacetamide-N-sulfonic acid triethylamine salt solution and a cyclizing agent formed by dissolving sulfur trioxide in a solvent are fully mixed through the Venturi reactor, injected to perform a sulfonation cyclization reaction under the action of a supported solid base heterogeneous catalyst to obtain a sulfonated cyclized product, then entered into the hydrolysis reactor to be hydrolyzed with a hydrolysis agent, and further a salt forming reaction is carried out to obtain a final acesulfame product. According to the present application, the reaction time of the cyclization reaction is greatly shortened, the probability of organic impurities remaining in the final acesulfame product is reduced, the purity of acesulfame is improved, the post-treatment process of acesulfame is simplified, and the production cost of acesulfame is reduced; and the yield of acesulfame is remarkably improved, and meanwhile, the reaction can be continuously carried out, suitable for large-scale industrial production, increasing the production efficiency of acesulfame.

The foregoing description is merely an overview of the technical solutions of the present application. In order to make the technical means of the present application clearer to be understood so as to be implemented according to the contents of the description, and in order to make the above and other objects, features and advantages of the present application more comprehensible, specific embodiments of the present application are described below.

### Detailed Description of Embodiments

Exemplary embodiments of the present application will be described in more detail below. It should be understood that the present application may be implemented in various forms and should not be limited by the embodiments set forth herein. Rather, these embodiments are provided to enable a more thorough understanding of the present application and can fully convey the scope of the present application to those skilled in the art.

The conception of the present application is to provide a method by combining a Venturi reaction, a flow reactor and a hydrolysis reactor against the problems of low yield and high impurity content of acesulfame caused by low reaction efficiency in the sulfonation cyclization step of the process of acesulfame preparation using a diketene-sulfur trioxide method in the prior art, effectively overcoming the above-mentioned technical defects by controlling reaction conditions. And the method significantly improves the yield of acesulfame on one hand and greatly reduces the content of organic impurities in the final acesulfame product on the other hand, and the present application can realize continuous reaction, suitable for large-scale industrial production.

The method for preparing acesulfame potassium provided by the present application is implemented by using a combined reactor, the combined reactor comprises a Venturi reactor, a flow reactor and a hydrolysis reactor, the outlet of a diffusion section of the Venturi reactor is connected to the inlet of the flow reactor, and the flow reactor is connected to the inlet of the hydrolysis reactor.

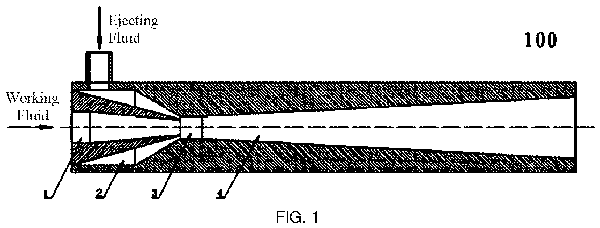

FIG. 1 is a schematic cross-sectional view of a Venturi reactor 100 according to the embodiment of the present disclosure, and it can be seen from FIG. 1 that the structural size in the Venturi reactor 100 has an important influence on the flow rate of the ejecting fluid and the mixing effect between the working fluid and the ejecting fluid. As shown in FIG. 1, the Venturi reactor is mainly composed of a nozzle 1, an air suction chamber 2, a mixing section 3, and a diffusion section 4.

The method for preparing acesulfame potassium provided by the present application uses the above-mentioned Venturi reactor to mix part of the raw materials, and differs from the prior art in that the outlet of the Venturi reactor of the present application is connected to the flow reactor, and the outlet of the flow reactor is connected to the inlet of the hydrolysis reactor.

The method for preparing acesulfame potassium provided by the present application at least comprises steps S110 to S140:
A mixing step S110: pressing the acetylacetamide-N-sulfonic acid triethylamine salt solution and the cyclizing agent solution from different inlets of the Venturi reactor, mixing in the mixing section and the diffusion section of the Venturi reactor, and injecting the obtained mixture into the flow reactor, wherein the cyclizing agent solution is formed by dissolving sulfur trioxide in the first organic solvent.

The nozzle 1 and the suction chamber 2 are inlets of the Venturi reactor such that the acetylacetamide-N-sulfonic acid triethylamine salt solution was entered into the Venturi reactor from one of the nozzle 1 and the suction chamber 2, and the cyclizing agent solution was entered from the other inlet of the Venturi reactor.

The principle of the Venturi reactor is that the working fluid with higher pressure is compressed and accelerated through the nozzle 1, the pressure energy is converted into kinetic energy, a high-speed low-pressure area is formed near the outlet of the nozzle 1, and then a pressure difference is formed between the inlet and the outlet of the air suction chamber 2. Under the action of the pressure difference, the ejecting fluid is drawn into the mixing section through the suction chamber 2. In the inlet of the mixing section 3, the momentum and energy of the working fluid and the ejecting fluid are exchanged with each other, due to the shearing action of the high-speed working fluid, the ejecting fluid sucked in can be crushed, promoting two-phase mutual mixing, increasing two-phase contact area, facilitating two-phase mass transfer, and thus increasing the reaction rate, then the two-phase fluid fully mixed is subjected to deceleration and pressurization through the diffusion section, the kinetic energy is converted into pressure energy, and it can be injected into the interior of the reactor connected to the outlet of the diffuser section 4. The acetylacetamide-N-sulfonic acid triethylamine salt solution and the cyclizing agent solution are uniformly mixed through the mixing section and the diffusion section of the Venturi reactor.

In this step, the acetylacetamide-N-sulfonic acid triethylamine salt has a good mixing effect with sulfur trioxide, and then the obtained mixture is injected into the flow reactor by the Venturi reactor.

The use of the Venturi reactor and the setting of the working parameters ensure the rapid mixing of the acetylacetamide-N-sulfonic acid triethylamine salt and sulfur trioxide, so that the mixing process can be completed within a very short time, such as 1-15 s, even within 1-3 s. The shortening of the mixing process can significantly shorten the reaction time of the whole reaction, and in this process, the probability of organic impurities remaining in the final acesulfame product is reduced, and the purity of acesulfame is improved.

A sulfonation cyclization step S120: subjecting the mixture passing through the flow reactor to a sulfonation cyclization reaction under the action of the supported solid base heterogeneous catalyst preset in the flow reactor, and entering the obtained sulfonated cyclized product into the hydrolysis reactor.

In the prior art, in the step of the sulfonation cyclization reaction, the acetylacetamide salt and sulfur trioxide are usually directly reacted, the reaction efficiency thereof is not high, it also causes some impurities with structure similar to acesulfame potassium to remain in the final acesulfame product, and the reaction yield cannot reach a satisfying level.

Unlike the prior art, the sulfonation cyclization step in the present application is completed under the action of a supported solid base heterogeneous catalyst. The catalyst can promote the forward progress of the sulfonation cyclization step, improving the yield of the sulfonated cyclization product, and further improving the yield of the final acesulfame product; and at the same time, the content of impurities in the product can also be reduced.

A hydrolysis step S130: subjecting the sulfonated cyclized product and a hydrolysis agent preset in a hydrolysis reactor to a hydrolysis reaction to obtain a hydrolysate solution;

The sulfonated cyclized product is subjected to a hydrolysis reaction with a hydrolysis agent preset in a hydrolysis reactor. The cyclized product is a precursor ASH of acesulfame after being hydrolyzed, which is different from that in a conventional process of hydrolysis reaction using an acidic solution. The hydrolysis agent used in the present application can shorten the hydrolysis time, and the reduction of the impurity content that may be generated by hydrolysis comes with the decrease of the hydrolysis time. In some embodiments of the present application, the hydrolysis agent is water or an ethanol aqueous solution, which significantly reduces the content of impurities in the cyclized product ASH, reduces the difficulty of subsequent purification of acesulfame, and reduces the cost of purification of acesulfame.

A salt forming step S140: adding potassium hydroxide into the organic phase of the hydrolysate solution, and carrying out a salt forming reaction to obtain acesulfame potassium.

After the sulfonated cyclized product is hydrolyzed, potassium hydroxide or a potassium hydroxide aqueous solution is usually used to react with the hydrolysate to obtain acesulfame potassium (ASK). The so-called salt forming reaction refers to the process of exchanging the cation of potassium hydroxide with the anion of the hydrolysate to produce the potassium salt of acesulfame.

In some embodiments of the present application, a salt forming agent may replace the traditional potassium hydroxide or potassium hydroxide aqueous solution to be used in the salt forming reaction, the inorganic impurities including, but are not limited to, potassium fluoride, potassium sulfate, and the like, are not dissolved in the ethanol solution of potassium ethoxide, and during the reaction of the salt forming agent and the sulfonated cyclization product in the organic phase, the inorganic impurities are dissolved in the aqueous phase without being brought into the final acesulfame potassium product. In addition, using the salt forming agent to neutralize the acid ASH corresponding to the acesulfame potassium, the possible acetylacetamide and acesulfame potassium chlorides are advantageously reduced in this process, thereby blocking the source of the inorganic impurities from the origin during the salt formation process, thereby improving the purity of the final product acesulfame potassium, simplifying the subsequent process for the crude potassium acesulfame product and reducing the cost for purifying the crude acesulfame potassium product. In some embodiments of the present application, the salt forming agent includes, but is not limited to, an ethanol solution of potassium hydroxide or an ethanol solution of potassium ethoxide.

Moreover, the combined reactor used in the present application realizes the continuous preparation of acesulfame potassium, suitable for large-scale process and production.

In conclusion, according to the present application, a Venturi reactor, a flow reactor and a hydrolysis reactor are combined for use to form a combined continuous reactor. In the combined reactor, an acetylacetamide-N-sulfonic acid triethylamine salt solution and a cyclizing agent formed by dissolving sulfur trioxide in a solvent are fully mixed through the Venturi reactor, injected to perform a sulfonation cyclization reaction under the action of a supported solid base heterogeneous catalyst to obtain a sulfonated cyclized product, then entered into the hydrolysis reactor to be hydrolyzed with a hydrolysis agent, and further a salt forming reaction is carried out to obtain a final acesulfame product. According to the present application, the reaction time of the cyclization reaction is greatly shortened, the probability of residual organic impurities in the final acesulfame product is reduced, the purity of acesulfame is improved, the post-treatment process of acesulfame is simplified, and the production cost of acesulfame is reduced; and the yield of acesulfame is remarkably improved, and meanwhile, the reaction can be continuously carried out, suitable for large-scale industrial production, increasing the production efficiency of acesulfame.

### Preparation Method of Acetylacetamide-N-Sulfonic Acid Triethylamine Salt Solution

In the present application, the source of the acetylacetamide-N-sulfonic acid triethylamine salt solution is not limited, and it can be prepared in reference to the prior art, or by the following method:
Adding triethylamine to a sulfamic acid solution, and carrying out an amination reaction to produce an ammonium sulfamate solution, adding diketene to the obtained ammonium sulfamate solution, and carrying out an acylation reaction under the action of a solid acidic catalyst to obtain an acetylacetamide-N-sulfonic acid triethylamine salt solution.

The preparation of the acetylacetamide-N-sulfonic acid triethylamine salt solution can be more meticulously divided into two small steps, first, the preparation of ammonium sulfamate, and then the reaction of ammonium sulfamate with diketene to prepare an intermediate, i.e. the acetylacetamide-N-sulfonic acid triethylamine salt.

The ammonium sulfamate is obtained by adding triethylamine to the sulfamic acid solution for amination reaction. Specifically, in some embodiments of the present application, the sulfamic acid is dissolved in a second organic solvent to prepare a first reaction solution; triethylamine is dissolved in a third organic solvent to prepare a second reaction solution, and the second reaction solution is added to the first reaction solution for amination reaction to form an ammonium sulfamate solution. The second organic solvent and the third organic solvent are inert organic solvents, such as dichloromethane, capable of providing a reaction environment for the amination reaction. Sulfamic acid and triethylamine perform an exothermic reaction, in the reaction process, the heat generated will vaporize part of dichloromethane, the vaporized dichloromethane leaving the reaction system will take the heat generated away, and further, the vaporized dichloromethane can also be recycled.

When the first reaction solution and the second reaction solution are mixed, the second reaction solution is preferably gradually dropped into the first reaction solution, so that the reaction can be more fully completed, not causing the concentration of part of the reactant to be too high and the reaction level to be too violent.

A specific embodiment for producing an ammonium sulfamate solution is given below, this embodiment is merely an exemplary description, and the specific production process of the ammonium sulfamate solution may be any one of the prior art. Sulfamic acid, a first dichloromethane, triethylamine and a second dichloromethane are weighed according to a preset dosage ratio, the first dichloromethane is added into a dried reaction kettle by opening the valve of the measuring tank for reactivity, and the stirring and the circulating pump is started; and the sulfamic acid is fed from the feeding hole. The circulating valve is closed, the feeding valve is opened, the mixed material in the dissolving kettle is sent to a dry synthesis kettle, circulating water is used for cooling, and the first reaction liquid is obtained when the temperature of the reaction kettle is reduced to room temperature (about 20-25 °C).

In a process the same as above, a second reaction solution of triethylamine dissolved in dichloromethane is obtained.

The second reaction solution is added dropwise to the first reaction solution, the pH value is 7-9 at the end of the dropwise addition, it stands for reaction for 1 hour, and the material after the above reaction is completed is an ammonium sulfamate solution.

After the sulfamic acid solution is obtained, the sulfamic acid solution is reacted with diketene to obtain an acetylacetamide-N-sulfonic acid triethylamine salt as an intermediate for preparing acesulfame.

In some embodiments of the present application, the molar ratio of sulfamic acid to the second organic solvent is 1: 3-5, preferably 1: 4; and the molar ratio of the diketene to the sulfur trioxide is 1: 2-15, preferably 1: 4.

In the prior art, the reaction between the sulfamic acid solution and diketene is carried out in the environment of acetic acid, the acetic acid is difficult to be completely removed from the final acesulfame product in the subsequent steps, and the acetic acid remaining in the acesulfame not only makes the acesulfame not good in color, but also brings odour.

In the present application, the use of a solid acidic catalyst instead of the conventional acetic acid effectively overcomes this problem. The solid acidic catalysis can provide sufficient acidic sites for acylation reaction, on one hand it can effectively catalyze the acylation reaction of the ammonium sulfamate salt and the diketene to be carried out smoothly, on the other hand the solid acidic catalysis will not be mixed into the reaction product, special treatment processes are not needed subsequently, and post-treatment economy and time cost are saved; and the adverse effect on the final product condition caused by the fact that acetic acid impurity without being removed remains in the final product in the prior art is avoided.

In order to increase the flash point of diketene, diketene is dissolved in a third solvent to prepare a third reaction solution, and the third solvent is an inert organic solvent, such as dichloromethane, capable of providing a reaction environment for an amination reaction. The reactor is filled with the solid acidic catalyst, the ammonium sulfamate solution and the third reaction solution are sequentially added to the reactor, and the reaction was carried out under a preset condition to form an acetylacetamide-N-sulfonic acid triethylamine salt solution as an intermediate solution.

In order to achieve the continuity of the reaction, in some embodiments of the present application, a continuous reactor may be selected to implement the present application, such as a fixed bed reactor, a continuous stirred tank reactor, or a microchannel reactor. A fixed bed reactor is taken as an example here to briefly describe the reaction process.

The fixed bed reactor is filled with a solid acidic catalyst as a catalyst, the fixed bed reactor is set to a preset working state, first the ammonium sulfamate solution is introduced into the fixed bed reactor, then the third reaction liquid is introduced into the fixed bed reactor in the same direction after the ammonium sulfamate solution flows normally, the contacting time of the two is within a preset condition by controlling the flow rate of the two, meanwhile the reaction temperature is also within a preset condition by controlling the heat exchange device of the fixed bed reactor, and the reaction can be ended when a preset reaction time is reached to obtain the acetylacetamide-N-sulfonic acid triethylamine salt solution product. Due to the characteristics of the fixed bed reactor, the present reaction can be continuously carried out, suitable for large-scale industrial production.

### Types and the Amount of the Solid Acidic Catalyst

Some solid acidic catalysts of the present application are molecular sieve catalysts or solid superacid catalysts. Wherein, the molecular sieve catalyst is an HZSM -5 molecular sieve and/or a Na-ZSM -5 molecular sieve; and the solid superacid catalyst is an SO₄²⁻/Fe₂O₃ type catalyst.

The amount of the solid acidic catalyst is not limited in the present application, and can be determined according to the type and specification of the selected reactor.

### Reaction Conditions for the Sulfonated Cyclization Reaction

In some embodiments of the present application, the reaction conditions for the sulfonation cyclization reaction are not limited, the reaction temperature of the sulfonation cyclization reaction is -20 to 40°C, in some other embodiments the reaction temperature is 10 to 30°C, and in some other further embodiments the reaction temperature is 15-25°C. The reaction time of the sulfonation cyclization reaction is 1 s to 30 min, and in other embodiments the reaction time is 2 s to 2 min. It is found through experiments that the sulfonation cyclization reaction can achieve a better effect at a relatively short reaction time under room temperature condition.

### Types of Solvents

In some embodiments of the present application, the first organic solvent is pentane, hexane, heptane or dichloromethane; and the second organic solvent is pentane, hexane, heptane or dichloromethane.

The first organic solvent and the second organic solvent may be the same or different, preferably the same, and in some embodiments of the present application, the first organic solvent and the second organic solvent are both dichloromethane.

### Types and the Amounts of Catalyst

In some embodiments of the present disclosure, the type of the supported solid base heterogeneous catalyst is not limited, and in some other embodiments, the supported solid base heterogeneous catalyst is a K₂O/-γAl₂O₃ catalyst, wherein K₂O is an active component, and γAl₂O₃ is a carrier. In some other further embodiments, organic silicon may also be added to the carrier to achieve a better beneficial effect.

### Working Pressure of Fluid

In some embodiments of the present application, the acetylacetamide-N-sulfonic acid triethylamine salt solution may be used as a working fluid, the cyclizing agent solution is used as an ejecting fluid, the working fluid is pressed in from the nozzle of the Venturi reactor, and the ejecting fluid is pressed in from the suction chamber of the Venturi reactor; and the pressure of the working fluid is controlled to be greater than the pressure of the ejecting fluid. In this way, the acetylacetamide-N-sulfonic acid triethylamine salt solution and the cyclizing agent solution can achieve a better mixing effect.

In some embodiments of the present application, the pressure of the working fluid and the ejecting fluid and the pressure difference between the two are not limited, and the pressure of the working fluid can be just controlled to be higher than the pressure of the ejecting fluid. In some other embodiments, the pressure of the working fluid is 0.4-1.6 MPa, the pressure of the ejecting fluid is 0.2-1.4 MPa, and the pressure of the working fluid is 0.2-0.4 MPa higher than the pressure of the ejecting fluid.

By controlling the pressure and pressure difference of the working fluid and the ejecting fluid, the purposes of controlling the mixing speed, the reaction speed and the dosage proportion of the working fluid and the ejection fluid are achieved, so that the two can be fully and rapidly mixed, and the dosage proportion of the working fluid and the ejecting fluid is controlled to be within a proper range.

### Conditions for Injecting the Sulfonated Cyclized Product into the Flow Reactor

In some embodiments of the present application, the conditions for injecting the sulfonated cyclized product into the flow reactor are not limited, and any condition in which the sulfonated cyclized product can be effectively and rapidly injected into the flow reactor is just suitable. In some other embodiments of the present application, the sulfonated cyclized product is ejected from the outlet of the diffusion section of the Venturi reactor at a pressure of 0.5-1.1 MPa and injected into the flow reactor.

### Types of the Hydrolysis Agent and Conditions for the Hydrolysis Reaction

In some embodiments of the present application, in the above-mentioned method, the hydrolysis agent is deionized water or an ethanol aqueous solution, preferably an aqueous solution of ethanol, wherein in the ethanol aqueous solution, the mass concentration of ethanol is preferably 60-85%. Through a large number of experiments, the inventors find that the using of a hydrolysis agent, especially an ethanol aqueous solution, and controlling the content of water in the hydrolysis agent, can obviously reduce the content of impurities in the acesulfame precursor ASH, reduce the difficulty of subsequent acesulfame purification, and cut the cost of acesulfame purification.

In the present application, the amount of the hydrolysis agent is not limited, the amount of the hydrolysis agent may be determined according to the amount of sulfur trioxide, and specifically, in some embodiments of the present application, the ratio of the amount of sulfur trioxide to the content of water in the hydrolysis agent is 1: 1-4, and in some other embodiments, 1: 1-1.5. That is, the amount of substance of water in the hydrolysis agent is preferably higher than the amount of substance of sulfur trioxide.

In some embodiments of the present application, the hydrolysis reaction conditions are not limited, and any condition in which requirements for hydrolysis reaction can be met is suitable; in some embodiments of the present application, the prior art can be referred; and in some other embodiments of the present application, in the hydrolysis step, the reaction temperature of the hydrolysis reaction is -20 to -10° C. That is, the hydrolysis reaction step of the present application is preferably performed at a lower temperature, and in the present application, the temperature control may be any one of the prior art, such as an air condensation technology, a circulating water condensation technology, a heat exchange plate, etc. Through repeated tests, it is found that-20 to -10°C is a suitable temperature for the hydrolysis reaction, if the reaction temperature is lower than -20° C, the hydrolysis may not be completed, causing the degree of hydrolysis not to be thorough, some cyclized products not to be converted, and the conversion rate of raw materials to be low, and if the reaction temperature is higher than -10° C, the reaction temperature is too high, so that the cyclized product is easy to decompose, not conducive to the development of the reaction towards the direction of hydrolysis reaction.

### Types of the Flow Reactor

In some embodiments of the present application, the type of the flow reactor is not limited, and the flow reactor is a fixed bed reactor or a tubular reactor. The fixed bed reactor or the tubular reactor may be any one of the prior art, and can also be appropriately improved according to the requirements of the reaction, which is not limited in the present application.

The amount of other reaction materials and other reaction conditions not mentioned above can be referred to the prior art, such as Chinese patent CN112142687 A.

### Preparation of Acetylacetamide-N-Sulfonic Acid Triethylamine Salt Solution:

An amination reaction step: 98 kg of sulfamic acid and a first dichloromethane are dissolved at a molar ratio of 1: 6, and the dissolution temperature is controlled to be about 20 to 25°C to obtain a dichloromethane solution of sulfamic acid, that is, the first reaction solution. The dissolution may be in a continuous mixing device or in a reaction vessel.

Triethylamine and a second dichloromethane are dissolved at a molar ratio of 1: 1, and the dissolving temperature is controlled to be 10 to 30°C to obtain a second reaction solution, wherein the mass ratio of the sulfamic acid to the triethylamine is 1: 1.2. The second reaction solution is gradually added dropwise into the reaction kettle where the first reaction solution is located for mixing and stirring, the temperature of the system is controlled to be 20 to 30° C, the system is controlled to be weakly alkaline, and an ammonium sulfamate solution was obtained after mixing well.

An acylation reaction step: diketene and a third dichloromethane in a molar ratio of 1: 1.5 are dissolved, and the dissolving temperature is controlled to be 10-20°C to obtain a third reaction solution.

After the solid superacid catalyst is fixed to the fixed bed reactor, the fixed bed reactor is started, and the circulating water is adjusted to make the circulating water work normally.

The ammonium sulfamate solution is introduced into the fixed bed reactor, after the ammonium sulfamate solution flows normally, the third reaction solution is introduced into the fixed bed reactor in the same direction as the ammonium sulfamate solution, and the amount of the ammonium sulfamate solution and the amount of the third reaction solution is controlled so that the molar ratio of sulfamic acid to diketene is 1: 1.1. After the reaction starts, the temperature of the cooling water is adjusted as low as possible, and the temperature of the reaction system is controlled to be 20 to 35° C. With the decline of the performance of the catalyst, the temperature can be slightly increased within the control range.

The flow rate of the ammonium sulfamate solution and diketene is controlled so that the reaction time is controlled to be about 100 seconds. The resulting target product acetylacetamide-N-sulfonic acid triethylamine salt solution is obtained.

The acetylacetamide-N-sulfonic acid triethylamine salt solution of each of the embodiments and comparisons in the present application is prepared by the above-mentioned method if not specifically described, and following special description if specifically described.

### Embodiment 1-Embodiment 2

A mixing step: an acetylacetamide-N-sulfonic acid triethylamine salt solution and a cyclizing agent solution were pressed in from different inlets of the Venturi reactor, and mixed in the mixing section and the diffusion section of the Venturi reactor , and then the obtained mixture was injected into the flow reactor, wherein the cyclizing agent solution was formed by dissolving sulfur trioxide in a first organic solvent.

A sulfonation cyclization step: the mixture passing through the flow reactor was subjected to a sulfonation cyclization reaction under the action of a supported solid base heterogeneous catalyst preset in the flow reactor, and the obtained sulfonated cyclized product was entered into the hydrolysis reactor; and the specific type of the supported solid base heterogeneous catalyst is referred to Table 1.

A hydrolysis step: the sulfonated cyclized product is subjected to a hydrolysis reaction with a 60% ethanol aqueous solution preset in the hydrolysis reactor to obtain a hydrolysate solution.

A salt forming step: potassium hydroxide was added into the organic phase of the hydrolysate solution to perform a salt forming reaction to obtain acesulfame potassium.

### Comparison 1

A mixing step: an acetylacetamide-N-sulfonic acid triethylamine salt solution and a cyclizing agent solution were pressed in from different inlets of the Venturi reactor, and mixed in the mixing section and the diffusion section of the Venturi reactor, and then the obtained mixture was injected into the flow reactor, wherein the cyclizing agent solution was formed by dissolving sulfur trioxide in a first organic solvent.

A sulfonation cyclization step: the mixture passing through the flow reactor was subjected to a sulfonation cyclization reaction in the flow reactor, and the obtained sulfonated cyclized product was entered into the hydrolysis reactor; and no catalyst was used in Comparison 1.

A hydrolysis step: the sulfonated cyclized product was subjected to a hydrolysis reaction with a 60% ethanol aqueous solution preset in the hydrolysis reactor to obtain a hydrolysate solution.

A salt forming step: potassium hydroxide was added into the organic phase of the hydrolysate solution to perform a salt forming reaction to obtain acesulfame potassium.

The difference between Embodiment 1, Embodiment 2 and Comparison 1 lies merely in whether a catalyst is used, and other conditions remain consistent. That is, except for the reaction conditions listed in Table 1, the reaction conditions of the other steps in each of the embodiments remain consistent.

**Table 1**

| Number | | Type of catalyst | Time of sulfonation cyclization reaction | Temperature of sulfonation cyclization reaction (°C) | Yield of acesulfame (%) | Content of organic impurity (ppm) |
|---|---|---|---|---|---|---|
| Embod iment 1 | Comparative Embodiment 1A | K₂O/- γAl₂O₃ | 1 sec | 25 | 13.1 | Not detected |
| | Embodiment 1B | K₂O/- γAl₂O₃ | 30 sec | 25 | 77.9 | Not detected |
| | Embodiment 1C | K₂O/- γAl₂O₃ | 1 min | 25 | 82.3 | 1.6 |
| | Embodiment 1D | K₂O/- γAl₂O₃ | 2 min | 15 | 83.1 | 4.9 |
| | Comparative Embodiment 1E | K₂O/- γAl₂O₃ | 10 min | 15 | 83.9 | 51.2 |
| | Comparative Embodiment 1F | K₂O/- γAl₂O₃ | 30 min | -20 | 79.2 | 77.9 |
| Embod iment 2 | Comparative Embodiment 2A | K₂O/- γAl₂O₃ added with organic silicon | 1 sec | 25 | 34.1 | Not detected |
| | Embodiment 2B | K₂O/- γAl₂O₃ added with organic silicon | 30 sec | 25 | 82.1 | Not detected |
| | Embodiment 2C | K₂O/- γAl₂O₃ added with organic silicon | 1 min | 25 | 84.9 | 1.2 |
| | Embodiment 2D | K₂O/- γAl₂O₃ added with organic silicon | 2 min | 15 | 83.1 | 3.9 |
| | Embodiment 2E | K₂O/- γAl₂O₃ added with organic silicon | 10 min | 15 | 83.3 | 19 |
| | Comparative Embodiment 2F | K₂O/- γAl₂O₃ added with organic silicon | 30 min | -20 | 74.9 | 48 |
| Compa rison | Comparison 1A | No catalyst | 1 min | 25 | 6.1 | 342 |
| | Comparison 1B | No catalyst | 30 min | 15 | 21.1 | 993 |
| | Comparison 1C | No catalyst | 60 min | 0 | 54.6 | 308 |
| | Comparison 1D | No catalyst | 240 min | -20 | 66.7 | 104 |

| | | | | | | |
|---|---|---|---|---|---|---|
| Note: the yield of acesulfame is calculated according to the mass of diketene involved in the reaction. | | | | | | |

As can be seen From Embodiment 1, Embodiment 2, and Comparison 1, when other reaction conditions are consistent, the yield of acesulfame reaches a higher yield under the condition in which the catalyst is used, compared with that under the condition in which the catalyst is not used, and the content of organic impurities in the product is very rare, and even in some embodiments, no organic impurities are detected.

As can be seen From Embodiment 1 and Embodiment 2, the shorter the sulfonation cyclization reaction time is, the less the organic impurity content in the product is, but the longer the reaction time is within 10 min, the higher the yield of acesulfame is, and therefore, the reaction time has a suitable reaction time of about 30 sec to 2 min.

In conclusion, according to the present application, a Venturi reactor, a flow reactor and a hydrolysis reactor are combined for use to form a set of combined continuous reactor. In the combined reactor, an acetylacetamide-N-sulfonic acid triethylamine salt solution and a cyclizing agent formed by dissolving sulfur trioxide in a solvent are fully mixed through the Venturi reactor, injected to perform a sulfonation cyclization reaction under the action of a supported solid base heterogeneous catalyst to obtain a sulfonated cyclized product, then entered into the hydrolysis reactor to be hydrolyzed with a hydrolysis agent, and further a salt forming reaction is carried out to obtain a final acesulfame product. According to the present application, the reaction time of the cyclization reaction is greatly shortened, the probability of organic impurities remaining in the final acesulfame product is reduced, the purity of acesulfame is improved, the post-treatment process of acesulfame is simplified, and the production cost of acesulfame is reduced; and the yield of acesulfame is remarkably improved, and meanwhile, the reaction can be continuously carried out, suitable for large-scale industrial production, increasing the production efficiency of acesulfame.

The above are merely specific embodiments of the present application, and under the above teachings of the present application, a person skilled in the art may perform other improvements or modifications on the basis of the above embodiments. It should be understood by those skilled in the art that the above-mentioned specific description is merely a better explanation of the purpose of the present application, and the scope of protection of the present application shall be subject to the scope of protection of the claims.

Moreover, those skilled in the art will understand that although some embodiments described herein include certain features rather than other features, combinations of features of different embodiments mean to be within the scope of the present application and form different embodiments. For example, in the following claims, any one of the claimed embodiments may be used in any combination.

## Claims

1. A method for preparing acesulfame potassium, wherein the method is implemented by using a combined reactor, the combined reactor comprises a Venturi reactor, a flow reactor and a hydrolysis reactor, the outlet of a diffusion section of the Venturi reactor is connected to the inlet of the flow reactor, and the flow reactor is connected to the inlet of the hydrolysis reactor; and the method comprises:
a mixing step: pressing an acetylacetamide-N-sulfonic acid triethylamine salt solution and a cyclizing agent solution from different inlets of the Venturi reactor, after mixing in a mixing section and the diffusion section of the Venturi reactor, injecting the obtained mixture into the flow reactor, wherein the cyclizing agent solution is formed by dissolving sulfur trioxide in a first organic solvent;
a sulfonation cyclization step: subjecting the mixture passing through the flow reactor to a sulfonation cyclization reaction under the action of a supported solid base heterogeneous catalyst preset in the flow reactor, and entering the resulting sulfonated cyclized product into the hydrolysis reactor;
a hydrolysis step: carrying out a hydrolysis reaction with the sulfonated cyclized product and a hydrolysis agent preset in the hydrolysis reactor to obtain a hydrolysate solution; and
a salt forming step: adding potassium hydroxide into the organic phase of the hydrolysate solution, and carrying out a salt forming reaction to obtain acesulfame potassium.

2. The method according to claim 1, wherein the acetylacetamide-N-sulfonic acid triethylamine salt solution is prepared by the following method:
dissolving sulfamic acid in a second organic solvent, then adding triethylamine into the second organic solvent, and carrying out an amination reaction to generate an ammonium sulfamate solution; and
adding diketene to the obtained ammonium sulfamate solution, and carrying out an acylation reaction to obtain an acetylacetamide-N-sulfonic acid triethylamine salt solution.

3. The method according to claim 2, wherein the molar ratio of sulfamic acid to the second organic solvent is 1: 3-5, preferably 1: 4;
the molar ratio of diketene to sulfur trioxide is 1: 2-15, preferably 1: 4;
the reaction temperature of the sulfonation cyclization reaction is-20 to 40° C, preferably 10 to 30° C, and more preferably 15 to 25° C; and the reaction time is 1 s-30 min, preferably 2 s -2 min.

4. The method according to claim 2, wherein the first organic solvent is pentane, hexane, heptane or dichloromethane; and
the second organic solvent is pentane, hexane, heptane or dichloromethane.

5. The method according to claim 1, wherein the supported solid base heterogeneous catalyst is a K₂O/-γAl₂O₃ catalyst, and preferably, the K₂O/-γAl₂O₃ catalyst is added with organic silicon.

6. The method according to claim 1, wherein the hydrolysis agent is an ethanol aqueous solution, wherein the mass fraction of ethanol in the ethanol aqueous solution is 60-85%.

7. The method according to claim 1, wherein the reaction temperature of the hydrolysis reaction is -10 to -20° C.

8. The method according to claim 1, wherein the acetylacetamide-N-sulfonic acid triethylamine salt solution is used as a working fluid, the working fluid is pressed in from the nozzle of the Venturi reactor, the cyclizing agent solution is used as an ejecting fluid, and the ejecting fluid is pressed in from the suction chamber of the Venturi reactor; and the pressure of the working fluid is controlled to be greater than the pressure of the ejecting fluid.

9. The method according to claim 8, wherein the pressure of the working fluid is 0.4-1.6 MPa, the pressure of the ejecting fluid is 0.2-1.4 MPa, and the pressure of the working fluid is 0.2-0.4MPa higher than the pressure of the ejecting fluid.

10. The method of claim 1, wherein the flow reactor is a fixed bed reactor or a tubular reactor.
